# EUROPEAN PATENT SPECIFICATION

(11) **EP 0 948 646 B1**
(45) Date of publication and mention of the grant of the patent: **07.02.2007**
(21) Application number: 97947328.7
(22) Date of filing: 05.11.1997
(51) Int. Cl.: C12Q 1/68, C12N 15/10

(54) **METHODS FOR IDENTIFYING GENES ESSENTIAL TO THE GROWTH OF AN ORGANISM**
VERFAHREN ZUR IDENTIFIZIERUNG VON ESSENTIELLEN GENEN FÜR DAS WACHSTUM EINES ORGANISMUS
PROCEDES D'IDENTIFICATION DE GENES INDISPENSABLES A LA CROISSANCE D'UN ORGANISME

(30) Priority: 06.11.1996 US 30159 P
(43) Date of publication of application: 13.10.1999
(73) Proprietor: SMITHKLINE BEECHAM CORPORATION, Philadelphia, PA 19101 (US)
(72) Inventor: MOONEY, Jeffrey, L., Limerick, PA 19468 (US); DeBOUCK, Christine, Marie, Wayne, PA 19087 (US)
(74) Representative: Connell, Anthony Christopher
(86) International application number: PCT/US1997/020004
(87) International publication number: WO 1998/020161

(56) References cited:
- EP-A- 0 467 349
- WO-A-97/10365
- WO-A-97/23642
- BENDER A. ET AL.: "MULTICOPY SUPPRESSION OF THE CDC24 BUDDING DEFECT IN YEAST BY CDC42 AND THREE NEWLY IDENTIFIED GENES INCLUDING THE RAS-RELATED GENE RSR1" PROCEEDINGS OF THE NATIONAL ACADEMY OF SCIENCES OF THE UNITED STATES, vol. 86, no. 24, 1989, pages 9976-9980, XP002282792 1989 ISSN: 0027-8424
- CHUN K.T. ET AL.: "THE IDENTIFICATION OF TRANSPOSON-TAGGED MUTATIONS IN ESSENTIAL GENES THAT AFFECT CELL MORPHOLOGY IN SACCHAROMYCES CEREVISIAE" GENETICS, vol. 142, no. 1, January 1996 (1996-01), pages 39-50, XP000995589 ISSN: 0016-6731
- LENNON G.G. ET AL.: "Hybridization analyses of arrayed cDNA libraries" TRENDS IN GENETICS, ELSEVIER SCIENCE PUBLISHERS B.V. AMSTERDAM, NL, vol. 7, no. 10, 1 October 1991 (1991-10-01), pages 314-317, XP002095118 ISSN: 0168-9525
- HENSEL M. ET AL.: "SIMULTANEOUS IDENTIFICATION OF BACTERIAL VIRULENCE GENES BY NEGATIVE SELECTION" SCIENCE, vol. 269, 21 July 1995 (1995-07-21), pages 400-403, XP000645478 ISSN: 0036-8075
- SHALON D. ET AL.: "A DNA MICROARRAY SYSTEM FOR ANALYZING COMPLEX DNA SAMPLES USING TWO-COLOR FLUORESCENT PROBE HYBRIDIZATION" GENOME RESEARCH, COLD SPRING HARBOR LABORATORY PRESS, US, vol. 6, no. 7, July 1996 (1996-07), pages 639-645, XP009007958 ISSN: 1088-9051
- GENOMICS, May 1996, Vol. 33, No. 3, SAPOLSKY et al., "Mapping Genomic Library Clones Using Oligonucleotide Arrays", pages 445-456.
- TRENDS IN GENETICS, October 1991, Vol. 7, No. 10, LENNON et al., "Hybridization Analyses of Arrayed cDNA Libraries", pages 314-317.

## Description

### FIELD OF THE INVENTION

The present invention relates to the use of high-density arrays or grids of genomic (or cDNA) libraries for the identification, sequencing and characterization of genes which are essential to the growth of an organism, and more specifically to a pathogen. The determination of these essential genes and the proteins encoded thereby is useful in the development of new therapies against such pathogens.

### BACKGROUND OF THE INVENTION

Identification, sequencing and characterization of genes is a major goal of modern scientific research. By identifying genes, determining their sequences and characterizing their biological function, it is possible to employ recombinant technology to produce large quantities of valuable gene products, e.g. proteins and peptides. Additionally, knowledge of gene sequences can provide a key to diagnosis, prognosis and treatment in a variety of infectious diseases and disease states in plants and animals which are characterized by inappropriate expression and/or repression of selected genes or by the influence of external factors, e.g., carcinogens or teratogens, on gene function.

Methods have been described for the identification of certain novel gene sequences, referred to as Expressed Sequence Tags (EST). Adams *et al., Science,* 1991, 252:1651-1656. A variety of techniques have also been described for identifying particular gene sequences on the basis of their gene products. For example, see International Patent Application No. WO91/07087, published May 30, 1991. In addition, methods have been described for the amplification of desired sequences. For example, see International Patent Application No. WO91/17271, published November 14, 1991.

Genes which are essential for the growth of an organism, however, have been difficult to identify in such a manner as to be easily recovered for future analysis. The most common methodology currently employed to identify essential genes is a multistep process involving the generation of a conditionally lethal mutant library followed by the screening of duplicate members under the appropriate permissive and non-permissive conditions. Candidate mutants are then transformed with a second, genomic library and the desired genes isolated by complementation of the mutant phenotype. The complementing plasmid is recovered, subcloned, and then retested. However, this procedure comprises multiple subcloning steps to identify and recover the desired genes thus making it both labor intensive and time consuming.

Accordingly, there exists a need for a more efficient method of identifying genes essential to the growth of an organism.

### SUMMARY OF THE INVENTION

In one aspect, the invention provides a method of identifying a gene or genes which are essential to the growth of an organism through the use of high density arrays or grids of genomic libraries. The method involves preparing a genomic library of a selected organism and providing a plurality of identical grids, each grid comprising a surface on which is immobilized at predefined regions on said surface a plurality of defined materials derived from the genomic library. The selected organism is then mutagenized, preferably by insertional mutagenesis, and grown in a test culture under a selected set of defined conditions. A control culture comprising the non-mutagenized selected organism is also grown under the same set of defined conditions. Surviving cells from the cultures are harvested and DNA from harvested cells of the mutagenized organism (test culture) and RNA, or DNA, from harvested cells of the non-mutagenized organism (control culture) are extracted and isolated. Labeled polynucleotide probes from the isolated DNA of the test culture and labeled polynucleotide probes from the isolated RNA (or DNA) of the control culture are then generated and hybridized to identical grids to produce a test hybridization pattern and a control hybridization pattern, respectively. Hybridization patterns on the grids are then compared to identify genes essential for growth of the selected organism. Essentiality of the identified gene for growth of the selected organism is then confirmed.

The method of the present invention may further comprise growing additional test cultures comprising the mutagenized organism and control cultures comprising the non-mutagenized organism under different sets of defined conditions. Labeled probes from the isolated DNA and RNA from these additional cultures are generated in the same fashion as previously described to produce test and control hybridization patterns for cultures grown under the different sets of defined conditions. Genes essential to the growth of the selected organism are then identified by comparing the hybridization patterns generated by mutagenized and non-mutagenized organisms grown under each of the different sets of defined conditions.

An additional aspect of the invention provides an isolated gene which is essential to the growth of an organism and is identified by one of the above methods.

Yet another aspect of the invention is an isolated protein produced by expression of the gene sequence identified above. Such proteins are useful in the development of therapeutic and diagnostic compositions, or as targets for drug development.

Yet another aspect of the invention is to identify broad spectrum antibiotics or antifungals which inhibit the expression of these essential genes.

In a related aspect, the present invention provides a method to identify conditionally lethal mutant genes of a selected organism by complementation with a non-mutagenized genomic library of the same organism. The method involves preparing a genomic library in either an integration vector, or in an expression vector, and providing a grid comprising a surface on which is immobilized at predefined regions on said surface a plurality of defined materials derived from the genomic library. The selected organism is then mutagenized, preferably by chemically induced point mutations, and grown (in a test culture) under permissive and non-permissive conditions to identify mutagenized organisms that contain conditionally lethal mutant genes. Organisms that contain conditionally lethal mutant genes are transformed with the prepared (i.e., non-mutagenized) genomic library and the transformed organisms, or cells, are grown under the same non-permissive conditions used to identify mutagenized organisms that contain the conditionally lethal mutant genes. Surviving cells are harvested and DNA is extracted and isolated. Labeled polynucleotide probes from the isolated DNA are then generated and hybridized to the grid to identify genes essential for growth of the selected organism.

Other objects, features, advantages and aspects of the present invention will become apparent to those of skill in the art from the following description. It should be understood, however, that the following description and the specific examples, while indicating preferred embodiments of the invention, are given by way of illustration only. Various changes and modifications within the scope of the disclosed invention will become readily apparent to those skilled in the art from reading the following description and from reading the other parts of the present disclosure.

### DETAILED DESCRIPTION OF THE INVENTION

The biochemical basis of many bacterial resistance mechanisms to antibiotics is now known. These mechanisms alone, or in concert, are responsible for the escalating problem of antibiotic resistance seen both in hospital and community acquired infection. The principle approach by researchers to overcome these problems has been to seek incremental improvements in existing drugs. Although these approaches contribute somewhat to the fight against infection by such resistant pathogens, new approaches are needed.

Methods have now been developed for identifying genes and gene products essential to the survival of an organism. Genes and gene products identified by these methods are useful as molecular targets for drug discovery. The methods of the present invention are useful in determining the effect of the total absence of a gene or gene product on the survival of an organism.

### I. Definitions

Several words and phrases used throughout this specification are defined as follows:

As used herein, the term "gene" refers to the genomic nucleotide sequence from which a cDNA sequence is derived. The term gene classically refers to the genomic sequence, which upon processing, can produce different cDNAs, e.g., by splicing events. However, for ease of reading, any full-length counterpart cDNA sequence will also be referred to by shorthand herein as gene.

By "gene product" it is meant any polypeptide sequence encoded by a gene. The term "genomic library" is meant to include, but is not limited to, plasmid libraries, PCR products from genomic libraries, cDNA libraries and known sequences. Methods for the construction of such libraries are well known by those skilled in the art. In a preferred embodiment of the present invention, a genomic library is constructed in a suicide vector. It is also preferred that the constructed library be adjusted to minimize the number of complete genes present in a single genomic insert to approximately one gene. Techniques for this adjustment are well known to the skilled artisan.

"Isolated" means altered "by the hand of man" from its natural state; i.e., that, if it occurs in nature, it has been changed or removed from its original environment, or both. For example, a naturally occurring polynucleotide or a polypeptide naturally present in a living animal in its natural state is not "isolated," but the same polynucleotide or polypeptide separated from the coexisting materials of its natural state is "isolated", as the term is employed herein. For example, with respect to polynucleotides, the term isolated means that it is separated from the chromosome and cell in which it naturally occurs.

By "organism" it is meant any single cell organism. Preferably this includes, but is not limited to, bacterium (including both gram negative and gram positive species), viruses and lower eukaryotic cells such as fungi, yeast, molds and simple multicellular organisms. Preferably, the organism is a pathogen.

The term "pathogen" is defined herein as any organism which is capable of infecting an animal or plant and replicating its nucleic acid sequences in the cells or tissue of that animal or plant. Such a pathogen is generally associated with a disease condition in the infected animal or plant. Such pathogens may include, but are not limited to, viruses, which replicate intra- or extra-cellularly, or other organisms such as bacteria, fungi or molds, which generally infect tissues or the blood. Certain pathogens are known to exist in sequential and distinguishable stages of development, e.g., latent stages, infective stages, and stages which cause symptomatic diseases. In these different states, the pathogen is anticipated to rely upon different genes as essential for survival or for pathogenicity.

As used herein, the term "solid support" refers to any known substrate which is useful for the immobilization of a plurality of defined materials derived from a genomic library by any available method to enable detectable hybridization of the immobilized polynucleotide sequences with other polynucleotides in the sample. Among a number of available solid supports, one desirable example is the supports described in International Patent Application No. WO91/07087, published May 30, 1991. Examples of other useful supports include, but are not limited to, nitrocellulose, nylon, glass, silica and Pall BIODYNE C. It is also anticipated that improvements yet to be made to conventional solid supports may also be employed in this invention.

The term "grid" means any generally two-dimensional structure on a solid support to which the defined materials of a genomic library are attached or immobilized.

As used herein, the term "predefined region" refers to a localized area on a surface of a solid support on which is immobilized one or multiple copies of a particular clone and which enables hybridization of that clone at the position, if hybridization of that clone to a sample polynucleotide occurs.

By "immobilized", it is meant to refer to the attachment of the genes to the solid support. Means of immobilization are known and conventional to those of skill in the art, and may depend on the type of support being used.

### II. Compositions of the Invention

The present invention is based upon the use of high density arrays or grids of genomic libraries as a means for rapidly identifying genes essential for the growth of an organism.

### A. Preparation of genomic libraries

For this analysis a random genomic library for the target organism is prepared. The genomic DNA is isolated using standard procedures for molecular biology such as those disclosed by Sambrook *et al.,* MOLECULAR CLONING, A LABORATORY MANUAL, 2nd Ed.; Cold Spring Harbor Laboratory Press, Cold Spring Harbor, New York, 1989. The genomic library is then constructed in accordance with procedures described by Fleischmann et al. *Science,* 1995, *269*:496-512. For the purposes of the present invention, a genomic library can comprise a plasmid library, PCR products from a genomic library, or known sequences. In one embodiment, a suicide vector is used for preparation of the genomic library. Examples of suicide vectors which may be used in the present invention are well known in the art. See, for example, Booker *et al. Lett. Appl. Microbiol.* 1995 *21*:292-297; Steinmeitz, M. and Richter, R. *Gene, 1994, 142*:79-83; Yu et al. *J. Bacteriol.* 1994 *176*:3627-34; and Quandt, J. and Hynes, M.F. *Gene,* 1993, *127*:15-21. In a preferred embodiment, a suicide vector containing the broad host range erythromycin (Erm) gene can be prepared in a commercially available plasmid such as pBluescript (pBS; Stratagene, La Jolla, CA). The Erm-gene is isolated as a Taq1 restriction fragment from the vector pE 194 (Hourinouchi, S. and Weisbaum, B. J. Bacteriology 1982, 150:804-812). The Erm containing fragment is ligated directly into Nael digested, CIP-treated pBS and transformed into HB101 cells. Transformants are screened by PCR to determine the presence of the Erm gene. Using this vector, two Erm positive isolates were confirmed by sequence analysis and designated pJMErmA4 and pJMErmD2. For library construction, genomic inserts are placed into the unique SmaI site present in the polylinker region. It is also preferred that the constructed library be adjusted to minimize the number of complete genes present in a single genomic insert. Techniques for making this adjustment to the library are well known to those skilled in the art.

### B. Preparation of Grid

A plurality of materials derived from the genomic library are gridded onto a surface of a solid support at predefined locations or regions, preferably at 6X coverage. By "plurality of materials derived from the genomic library" it is meant to include, but is not limited to, bacterium containing individual clones spotted onto and grown on a surface of the solid support at predefined locations or regions; or plasmid clones isolated from said library, PCR products derived from the inserts from the plasmid clones, or oligonucleotides derived from sequencing of the plasmid clones, which are immobilized to the surface of the solid support at predefined locations or regions.

Numerous conventional methods are employed for immobilizing these materials to surfaces of a variety of solid supports. See, e.g., Affinity Techniques, Enzyme Purification: Part P, Methods in Enzymology, Vol. 34, ed. W.B. Jakoby, M. Wilcheck, Acad. Press, NY (1971); Immobilized Biochemicals and Affinity Chromatography, Advances in Experimental Medicine and Biology, Vol. 42, ed. R. Dunlap, Plenum Press, NY (1974); U.S. Patent 4,762,881; U.S. Patent No. 4,542,102; European Patent Publication No. 391,608 (October 10, 1990); or U.S. Patent No. 4,992,127 (November 21, 1989).

One desirable method for attaching these materials to a solid support is described in International Application No. PCT/US90/06607 (published May 30, 1991). Briefly, this method involves forming predefined regions on a surface of a solid support, where the predefined regions are capable of immobilizing the materials. The method makes use of binding substrates attached to the surface which enable selective activation of the predefined regions. Upon activation, these binding substances become capable of binding and immobilizing the materials derived from the genomic library.

Any of the known solid substrates suitable for binding nucleotide sequences at predefined regions on the surface thereof for hybridization and methods for attaching nucleotide sequences thereto may be employed by one of skill in the art according to the invention. Similarly, known conventional methods for making hybridization of the immobilized materials detectable, e.g., fluorescence, radioactivity, photoactivation, biotinylation, energy transfer, solid state circuitry, and the like may be used in this invention.

### C. Preparation and Growth of Mutagenized Organism

The organism of interest is mutagenized by transfection with either a randomly integrating transposon or similar insertional or transposable elements of known sequence (e.g., Tn, IS, phage Mu, Ty element) or with a constructed suicide vector and allowed to grow under a selected set of defined conditions.

### III. The Methods of the Invention

### A. Identification of Genes

The present invention employs the compositions described above in methods for identifying genes which are essential to the growth of an organism. These methods may be employed to detect such genes, regardless of the state of knowledge about the function of the gene.

In one embodiment, a gene or genes which are essential to the growth of a selected organism are identified through the use of two or more identical high density arrays or grids of genomic libraries prepared from the selected organism. For this analysis, at least two identical high density grids or arrays are prepared. Each grid is prepared from a random genomic library for a selected organism, preferably in a suicide vector. A plurality of defined materials derived from the genomic library are then gridded onto a solid support, preferably at 6X coverage. The insert size of this library is adjusted to minimize the number of complete genes that might be present in a single insert. In a preferred embodiment, the target insert size is one complete gene. For bacteria, the average length of a complete gene is approximately 1 kb.

The selected organism is mutagenized by transfection with either a randomly integrating transposon or similar insertional or transposable element of known sequence, such as Tn, IS, Ty element or phage Mu, or with the constructed suicide vector. The mutagenized selected organism is then cultured under a selected set of defined *in vitro* or *in vivo* conditions to produce a test culture. In addition, a non-mutagenized selected organism is also cultured under the same set of defined conditions to produce a control culture. By "defined conditions" it is meant, but is not limited to, standard *in vitro* culture conditions recognized as normal (i.e., non-pathogenic) for a selected organism, as well as *in vitro* conditions which reflect or mimic *in vivo* pathogenic settings (conditions) such as heat shock, auxotrophic, osmotic shock, antibiotic or drug selection/addition, varied carbon sources, and aerobic or anaerobic conditions, and *in vivo,* pathogenic conditions. Preferably, such conditions are predetermined to allow maximum growth of the non-mutagenized organism. The surviving cells are then harvested. Harvesting can be performed during various growth stages of the cells to ascertain the essentiality of a particular gene during different stages of growth. For example, harvesting can be performed during early logarithmic growth, late logarithmic growth, stationary phase growth or late stationary growth. RNA (or DNA) is then extracted and isolated from the harvested non-mutagenized cells of the control culture, while DNA is extracted and isolated from the mutagenized cells of the test culture using standard methodologies well known to those skilled in the art.

RNA (or DNA) extracted from the non-mutagenized cells of the control culture and DNA extracted from the mutagenized cells of the test culture are then used to generate labeled probes. The extracted, isolated DNA of the test culture serves as templates in primer extension reactions using oligonucleotide primers directed against a transposon/integrated vector sequence and which extends into the neighboring (i.e., flanking) nucleic acid sequence of the (genomic) DNA. Such primers will vary depending upon the mutagenesis/vector system employed. For example, in one embodiment, where the libraries constructed in the pJMErmA4 or pJMErmD2 vectors are used for both gridding and mutagenesis, primers designated against sequences which flank the SmaI cloning site are used. Examples of such primers include, but are not limited to:
5'-AATTAACCCTCACTAAAGGGAACA-3' (SEQ ID NO:1);
5'-TGTTCCCTTTAGTGAGGGTTAATT-3' (SEQ ID NO:2);
5'-GTAATACGACTCACGGAGGGGCGA-3' (SEQ ID NO:3); and
5'-ACGCCCCTCCGTGAGTCGTATTAG-3' (SEQ ID NO:4).
The extension reactions are performed using detectably labeled, i.e. radio- or fluorescent dye-labeled or biotinylated, nucleotides and controlled so that the extension products average approximately 200 base pairs (bp) in length. A number of methods exist for generating the primer extension products. In one embodiment, primer extension reactions are performed under the following conditions: A sample containing 15 pmoles of appropriate primer or primers, 5 pmoles extracted DNA, 30 mM Tris-HCl (pH 7.5), 50 mM NaCl, 1 mM DTT, 0.1 mM dATP, 0.1 mM ³²P-dCTP, 0.1 mM dGTP, 0.1 mM dTTP, 0.25 mM ddATP, 0.25 mM ddCTP, 0.25 mM ddGTP and water to 135 µl is prepared. This sample is then incubated at 75°C for 15 minutes; 50°C for 30 minutes and 37°C for 15 minutes. Klenow polymerase (75 units in a total volume of 15 µl) is then added and the sample is incubated for 30 minutes at 37°C. EDTA to 20 mM is the added. The sample is then extracted 1 time each with phenol, chloroform and isoamyl alcohol, followed by a second extraction with chloroform and isoamyl alcohol. The product is then precipitated with ethanol.

When RNA (or DNA) from the non-mutagenized organism is used to.generate the probes, isolated RNA (or DNA) is labeled according to standard methods using random primers, preferably hexamers, and reverse transcriptase. Such methods are routinely performed by those skilled in the art.

These labeled products are then used as hybridization probes against the identical high density grids. Labeled probes prepared from DNA extracted from mutagenized cells of the test culture are hybridized to one identical grid, while labeled probes from the RNA extracted from the non-mutagenized cells of the control culture are hybridized to a second identical grid. The generated test hybridization patterns and control hybridization patterns are then compared. Genes essential for the growth of the selected organism are identified by determining differences at the predefined regions of the grids between the test hybridization pattern and the control hybridization pattern grown under the selected set of defined conditions

Alternatively, additional test cultures comprising the mutagenized selected organism and control cultures comprising the non-mutagenized selected organism are grown under different sets of defined *in vitro* and *in vivo* conditions. Hybridization patterns for labeled polynucleotide probes prepared from DNA of the additional test cultures and RNA of the additional control cultures are then generated in accordance with procedures described herein. Genes essential to the growth of the organism are then identified by comparing the hybridization patterns of the test and control cultures for each set of defined conditions with each other. In one embodiment, genes essential to the growth of the organism will be those common to all of the hybridization patterns for all the cells. In another embodiment, genes essential for growth of a selected organism will hybridize under one set of growth conditions and will not hybridize under a different set of growth conditions.

In another embodiment, a pool of conditionally lethal mutants of the organism can be generated and transformed with a second (genomic) library constructed in a transposon/integration based vector. Transformants are reselected under the original conditionally lethal conditions and the rescued, surviving isolates used for probe generation and hybridization analysis as described above. For example, a temperature sensitive (ts) mutant library is prepared according to standard procedures and screened under permissive vs. non-permissive conditions to identify conditionally lethal mutants. The identified conditionally lethal ts mutants are pooled and transformed with a second, genomic library constructed in a transposon/integration based vector containing both a conditional and a selectable marker system. Examples of vectors for this second library include, but are not limited to, pMAK705 (Bloomfield et al. *Mol. Microbiol.* 1991 *5*:1447-1457) and pG+host5 (Biswas et al. *J. Bacteriol.* 1993, 175:3628-3635). The resulting transformants are retested or grown under the original temperature selection for lethality/essentiality. Survivors represent isolates containing integrated vector plus complementing genomic sequences. DNA from these survivors is then isolated and probes are generated as described in the preceding paragraphs, whereby hybridizing clones identify essential genes of interest.

In yet another embodiment, a conditionally lethal mutant library is prepared according to standard procedures, is constructed in an expression vector, and transformed with a selectable, genomic library. The genomic library is constructed using standard molecular biology techniques such that expression of the inserted genomic DNA is under control of vector-located promoter sequences, and preferably contains selectable and conditional markers. Examples of vectors containing inducible promoter systems include, but are not limited to, pFL10 (Lopez de Felipe et al. *FEMS Microbiol. Lett.* 1994, *122*: 289-295) and pUB110 (Zyprian, E. and Matzura, H. *DNA* 1986, *5*:219-225). In this embodiment, temperature sensitive lethal mutants are screened under temperature sensitive selection and under induction conditions for the vector-located promoter sequences. Surviving isolates represent clones where transcription of the exogenous plasmid insert complements the mutant phenotype. Probes are generated against the plasmid inserts and hybridized against the grids.

Essentiality of the gene to the organism is confirmed by inactivating the identified gene in the selected organism, preferably using a single gene disruption procedure such as a knock out experiment, and culturing the selected organism under the same defined conditions.

Clones identified by the methods of the instant invention can be used directly for sequence analysis and for knockout experiments to confirm their essentiality to the growth of the organism. Alternatively, a gene sequence from the identified clone can be subcloned into a suitable vector for knockout experiments as is common in the art. Sequence analysis is performed using standard methodologies well known to those skilled in the art. Initial sequencing may be performed using the M13 universal forward and universal reverse sequencing primers which flank the multiple cloning site of the vector. The resulting sequences are analyzed using conventional computer programs. Results of said analysis are used in determining the potential usefulness of the individual clones as antimicrobial targets.

For knockout experiments, plasmid DNA from the identified isolates is purified and transformed in a non-mutagenized organism using standard molecular biology techniques. The transformed cells are grown under antibiotic selection for the vector sequence. Surviving cells represent site-specific insertional events into genes which are not essential for growth since knockout of an essential gene would result in no viable transformants. DNA is isolated from the surviving cells and used as a template to generate probes in accordance with previously described procedures and the grids reprobed for analysis. Additional gene knockout experiments can be performed in accordance with procedures described by, for example, Guiterrez et al. *J. Bacteriol.* 1996*178*:4166-4175. Gene knockout experiments thus provide information on the effect of the total absence of the gene product.

### B. Other Methods of the Invention

As is obvious to one of skill in the art upon reading this disclosure, the compositions and methods of the invention may also be used for other similar purposes. For example, in one embodiment, this method can be used to monitor the effect of potential drugs on essential gene expression, both in laboratories and during clinical trials with animals, especially humans. Because the method can be readily adapted by altering growth conditions or the stage at which the cells are harvested, it can essentially be employed to identify essential genes of any organism, at any stage of development, and under the influence of any factor which can affect gene expression.

### IV. The Genes and Proteins Identified

Application of the compositions and methods of this invention as above described also provides other compositions, such as any isolated gene sequence which is essential to the growth of an organism. Another embodiment of this invention is any isolated pathogen gene sequence found to be essential to the survival of the pathogen in a host. Similarly, an embodiment of the invention is any gene sequence identified by the methods described therein.

These gene sequences may be employed in conventional methods to produce isolated proteins encoded thereby. To produce a protein of this invention, the DNA sequences of a desired gene invention or portions thereof identified by use of the methods of this invention are inserted into a suitable expression system. In a preferred embodiment, a recombinant molecule or vector is constructed in which the polynucleotide sequence encoding the protein is operably linked to a heterologous expression control sequence permitting expression of the human protein. Numerous types of appropriate expression vectors and host cell systems are known in the art for mammalian (including human), insect, yeast, fungal and bacterial expression.

The transfection of these vectors into appropriate host cells, whether mammalian, bacterial, fungal or insect, or into appropriate viruses, results in expression of the selected proteins. Suitable host cells, cell lines for transfection and viruses, as well as methods for construction and transfection of such host cells and viruses are well-known. Suitable methods for transfection, culture, amplification, screening and product production and purification are also known in the art.

In one embodiment, the essential genes and proteins encoded thereby which have been identified by this invention can be employed as diagnostic compositions useful in the diagnosis of a disease or infection by conventional diagnostic assays. For example, a diagnostic reagent can be developed which detectably targets a gene sequence or protein of this invention in a biological sample of an animal. Such a reagent may be a complementary nucleotide sequence, an antibody (monoclonal, recombinant or polyclonal), or a chemically derived agonist or antagonist. Alternatively, the essential genes of this invention and proteins encoded thereby, fragments of the same, or complementary sequences thereto, may themselves be used as diagnostic reagents. These reagents may optionally be detectably labeled, for example, with a radioisotope or colorimetric enzyme. Selection of an appropriate diagnostic assay format and detection system is within the skill of the art and may readily be chosen without requiring additional explanation by resort to the wealth of art in the diagnostic area.

Additionally, genes and proteins identified according to this invention may be used therapeutically. For example, genes identified as essential in accordance with this method and proteins encoded thereby may serve as targets for the screening and development of natural or synthetic chemical compounds which have utility as therapeutic drugs for the treatment of disease states associated with the organism. As an example, a compound capable of binding to a protein encoded by an essential gene thus preventing its biological activity may be useful as a drug component preventing diseases or disorders resulting from the growth of a particular organism. Alternatively, compounds which inhibit expression of an essential gene are also believed to be useful therapeutically. In addition, compounds which enhance the expression of genes essential to the growth of an organism may also be used to promote the growth of a particular organism.

Conventional assays and techniques may be used for screening and development of such drugs. For example, a method for identifying compounds which specifically bind to or inhibit proteins encoded by these gene sequences can include simply the steps of contacting a selected protein or gene product with a test compound to permit binding of the test compound to the protein; and determining the amount of test compound, if any, which is bound to the protein. Such a method may involve the incubation of the test compound and the protein immobilized on a solid support. Still other conventional methods of drug screening can involve employing a suitable computer program to determine compounds having similar or complementary structure to that of the gene product or portions thereof and screening those compounds for competitive binding to the protein. Identified compounds may be incorporated into an appropriate therapeutic formulation, alone or in combination with other active ingredients. Methods of formulating therapeutic compositions, as well as suitable pharmaceutical carriers, and the like are well known to those of skill in the art.

Accordingly, through use of such methods, the present invention is believed to provide compounds capable of interacting with these genes, or encoded proteins or fragments thereof, and either enhancing or decreasing the biological activity, as desired. Thus, these compounds are also encompassed by this invention.

Numerous modifications and variations of the present invention are included in the above-identified specification and are expected to be obvious to one of skill in the art. Such modifications and alterations to the compositions and processes of the present invention are believed to be encompassed in the scope of the claims appended hereto.

## Claims

1. A method of identifying genes essential to growth of single celled organisms including bacteria, viruses, and fungi comprising:
(a) preparing a genomic library of the single celled organism;
(b) providing a plurality of identical grids, each grid comprising a surface on which is immobilized at predefined regions on said surface a plurality of materials derived from the genomic library defined in (a);
(c) mutagenizing the single celled organism by transfection with *(i)* a randomly integrating transposon or *(ii)* a similar insertional or transposable element of known sequence or *(iii)* with a constructed suicide vector;
(d) growing a test culture comprising the mutagenized single celled organism and a control culture comprising non-mutagenized single celled organisms under a set of defined conditions;
(e) harvesting surviving cells from the cultures;
(f) extracting and isolating DNA from harvested cells of the test culture;
(g) extracting and isolating RNA or DNA from harvested cells of the control culture;
(h) generating labeled polynucleotide probes using an oligonucleotide primer extension reaction directed against *(i)* the randomly integrating transposon or *(ii)* similar insertional or transposable element of known sequence or *(iii)* the constructed suicide vector, wherein the reaction extends into flanking genomic DNA;
(i) generating labeled polynucleotide probes from the isolated RNA or DNA of the control culture;
(j) hybridizing the labeled probes generated from the isolated DNA of the test culture to a first identical grid to produce a test hybridization pattern;
(k) hybridizing the labeled probes generated from the isolated RNA or DNA of the control culture to a second identical grid to produce a control hybridization pattern;
(l) comparing the hybridization patterns to identify genes essential for growth in the single celled organism; and
(m) confirming that said identified gene is essential for growth of the single celled organism.

2. The method of claim 1 wherein the set of defined conditions of step (d) comprises standard non-pathogenic *in vitro* culture conditions for the single celled organism.

3. The method of claim 1 wherein the set of defined conditions of step (d) comprises *in vitro* conditions which reflect or mimic *in vivo* conditions, selected from the group of: aerobic or anaerobic conditions, auxotrophic, heat-shock, osmotic-shock, addition or presence of antibiotics or drugs, carbon source variations, and *in vivo* pathogenic conditions.

4. The method of claim 1 wherein the harvesting of surviving cells of step (e) is performed (i) during early logarithmic growth, (ii) during late logarithmic growth, (iii) during stationary phase growth, or (iv) during late stationary phase growth.

5. The method of claim 1 wherein:
step (d) further comprises growing additional test and control cultures under a different set of defined conditions; and
step (1) comprises comparing test and control hybridization patterns from the cells grown under the different sets of defined conditions.

6. The method of claim 6 wherein genes essential to the selected organism are identified by determining (i) identical hybridization patterns for all of the cells grown under the different sets of defined conditions, or (ii) differences between the test and control hybridization patterns for cells grown under the different sets of defined conditions.

7. A method of identifying genes essential to growth of single celled organisms including bacteria, viruses, and fungi by identifying conditionally lethal mutant genes, which comprises:
(a) preparing a genomic library of the single celled organism : (*i*) in an integration vector; or (*ii*) in an expression vector;
(b) providing a grid comprising a surface on which is immobilized at predefined regions on said surface a plurality of materials derived from the genomic library defined in (a);
(c) mutagenizing the single celled organism: by transfection with *(i)* a randomly integrating transposon or *(ii)* a similar insertional or transposable element of known sequence or *(iii)* with a constructed suicide vector;
(d) growing the mutagenized single celled organisms under permissive and non-permissive conditions to identify mutagenized single celled organisms, containing conditionally lethal mutant genes, to provide a pool of conditionally lethal mutant genes;
(e) transforming the pool of single celled organisms containing said conditionally lethal mutant genes with the genomic library of step (a);
(f) growing the transformed cells under the same non-permissive conditions as step (d) to identify transformed cells in which conditionally lethal mutant genes have been complemented with vector plus complementing genomic sequences;
(g) harvesting surviving cells;
(h) extracting and isolating DNA from the harvested cells;
(i) generating labeled polynucleotide probes wherein an oligonucleotide primer extension reaction directed against *(i)* the randomly integrating transposon or *(ii)* similar insertional or transposable element of known sequence or *(iii)* the constructed suicide vector, and the reaction extends into flanking genomic DNA; and
(j) hybridizing the labeled probes generated from the isolated DNA to a grid according to step (b), whereby such probes that hybridize to the grid identify genes essential for growth of the single celled organism.

8. The method of claim 1 or 7 wherein the plurality of materials derived from the genomic library of step (b) comprises bacteria containing individual clones spotted onto and grown on a surface of the solid support at predefined locations or regions, or plasmid clones isolated from said library, PCR products derived from the inserts from the plasmid clones, or oligonucleotides derived from sequencing of the plasmid clones, which are immobilized to the surface of the solid support at predefined locations or regions.

## Patentansprüche

1. Verfahren zum Identifizieren von Genen, die wesentlich für das Wachstum von einzelligen Organismen sind, einschließlich Bakterien, Viren und Pilzen, umfassend:
(a) Herstellen einer genomischen Bibliothek des einzelligen Organismus;
(b) Bereitstellen einer Mehrzahl von identischen Gittern, wobei jedes Gitter eine Oberfläche umfaßt, auf der in vordefinierten Regionen auf der Oberfläche eine Mehrzahl von Materialien immobilisiert ist, die aus der in (a) definierten genomischen Bibliothek stammen;
(c) Mutagenisieren des einzelligen Organismus durch Transfektion mit (i) einem zufällig integrierenden Transposon oder (ii) einem ähnlichen Insertions- oder transponiblen Element bekannter Sequenz oder (iii) einem konstruierten Suizidvektor;
(d) Kultivieren einer Testkultur, die den mutagenisierten einzelligen Organismus und eine Kontrollkultur umfaßt, die nicht-mutagenisierte einzellige Organismen umfaßt, unter einem Satz definierter Bedingungen;
(e) Ernten überlebender Zellen aus den Kulturen;
(f) Extrahieren und Isolieren von DNA aus geernteten Zellen der Testkultur;
(g) Extrahieren und Isolieren von RNA oder DNA aus geernteten Zellen der Kontrollkultur;
(h) Erzeugen markierter Polynukleotidsonden unter Verwendung einer Oligonukleotidprimer-Verlängerungsreaktion, die gegen (i) das zufällig integrierende Transposon oder (ii) das ähnliche Insertions- oder transponible Element bekannter Sequenz oder (iii) den konstruierten Suizidvektor gerichtet ist, worin sich die Reaktion in flankierende genomische DNA erstreckt;
(i) Erzeugen markierter Polynukleotidsonden aus der isolierten RNA oder DNA der Kontrollkultur;
(j) Hybridisieren der aus der isolierten DNA der Testkultur erzeugten markierten Sonden mit einem ersten identischen Gitter zur Erzeugung eines Testhybridisierungsmusters;
(k) Hybridisieren der aus der isolierten RNA oder DNA der Kontrollkultur erzeugten markierten Sonden mit einem zweiten identischen Gitter zur Erzeugung eines Kontrollhybridisierungsmusters;
(l) Vergleichen der Hybridisierungsmuster zur Identifizierung von Genen, die wesentlich für das Wachstum im einzelligen Organismus sind; und
(m) Bestätigen, daß das identifizierte Gen wesentlich für das Wachstum des einzelligen Organismus ist.

2. Verfahren gemäß Anspruch 1, worin der Satz definierter Bedingungen in Schritt (d) standardmäßige nichtpathogene Kulturbedingungen in vitro für den einzelligen Organismus umfaßt.

3. Verfahren gemäß Anspruch 1, worin der Satz definierter Bedingungen in Schritt (d) in-vitro-Bedingungen umfaßt, die in-vivo-Bedingungen widerspiegeln oder nachahmen, ausgewählt aus der Gruppe aus: aeroben oder anaeroben Bedingungen, auxotrophen Bedingungen, Hitzeschock, osmotischem Schock, Zugabe oder Gegenwart von Antibiotika oder Wirkstoffen, Variationen der Kohlenstoffquelle, und pathogenen Bedingungen in vivo.

4. Verfahren gemäß Anspruch 1, worin das Ernten der überlebenden Zellen in Schritt (e) durchgeführt wird (i) während des frühen logarithmischen Wachstums, (ii) während des späten logarithmischen Wachstums, (iii) während des Wachstums der stationären Phase oder (iv) während des Wachstums der späten stationären Phase.

5. Verfahren gemäß Anspruch 1, worin:
Schritt (d) ferner das Kultivieren zusätzlicher Test- und Kontrollkulturen unter einem unterschiedlichen Satz definierter Bedingungen umfaßt; und
Schritt (1) das Vergleichen von Test- und Kontrollhybridisierungsmustern aus den unter den unterschiedlichen Sätzen definierter Bedingungen kultivierten Zellen umfaßt.

6. Verfahren gemäß Anspruch 1, worin Gene, die für den ausgewählten Organismus wesentlich sind, identifiziert werden durch Bestimmung (i) identischer Hybridisierungsmuster für alle der unter den unterschiedlichen Sätzen definierter Bedingungen kultivierten Zellen oder (ii) von Unterschieden zwischen den Test- und Kontrollhybridisierungsmustern für unter den unterschiedlichen Sätzen definierter Bedingungen kultivierte Zellen.

7. Verfahren zum Identifizieren von Genen, die wesentlich für das Wachstum einzelliger Organismen sind, einschließlich Bakterien, Viren und Pilzen, durch Identifizieren konditional letaler mutierter Gene, welches folgendes umfaßt:
(a) Herstellen einer genomischen Bibliothek des einzelligen Organismus: (i) in einem Integrationsvektor; oder (ii) in einem Expressionsvektor;
(b) Bereitstellen eines Gitters, das eine Oberfläche umfaßt, auf der in vordefinierten Regionen auf der Oberfläche eine Mehrzahl von Materialien immobilisiert ist, die aus der in (a) definierten genomischen Bibliothek stammen;
(c) Mutagenisieren des einzelligen Organismus: durch Transfektion mit (i) einem zufällig integrierenden Transposon oder (ii) einem ähnlichen Insertions- oder transponiblen Element bekannter Sequenz oder (iii) einem konstruierten Suizidvektor;
(d) Kultivieren der mutagenisierten einzelligen Organismen unter permissiven und nicht-permissiven Bedingungen zur Identifizierung mutagenisierter einzelliger Organismen, die konditional letale mutierte Gene enthalten, um einen Pool konditional letaler mutierter Gene bereitzustellen;
(e) Transformieren des Pools einzelliger Organismen, die die konditional letalen mutierten Gene enthalten, mit der genomischen Bibliothek aus Schritt (a);
(f) Kultivieren der transformierten Zellen unter den gleichen nicht-permissiven Bedingungen wie in Schritt (d) zur Identifizierung transformierter Zellen, in denen konditional letale mutierte Gene mit Vektor plus komplementierenden genomischen Sequenzen komplementiert wurden;
(g) Ernten überlebender Zellen;
(h) Extrahieren und Isolieren von DNA aus den geernteten Zellen;
(i) Erzeugen markierter Polynukleotidsonden unter Verwendung einer Oligonukleotidprimer-Verlängerungsreaktion, die gegen (i) das zufällig integrierende Transposon oder (ii) das ähnliche Insertions- oder transponible Element bekannter Sequenz oder (iii) den konstruierten Suizidvektor gerichtet ist, worin sich die Reaktion in flankierende genomische DNA erstreckt; und
(j) Hybridisieren der aus der isolierten DNA erzeugten markierten Sonden mit einem Gitter gemäß Schritt (b), wodurch solche Sonden, die mit dem Gitter hybridisieren, Gene identifizieren, die wesentlich für das Wachstum des einzelligen Organismus sind.

8. Verfahren gemäß Anspruch 1 oder 7, worin die Mehrzahl von Materialien, die aus der genomischen Bibliothek aus Schritt (b) stammen, Bakterien umfaßt, die individuelle Klone enthalten, die auf eine Oberfläche des festen Trägers in vordefinierten Orten oder Regionen getüpfelt und darauf kultiviert werden, oder Plasmidklone, die aus der Bibliothek isoliert sind, PCR-Produkte, die aus den Inserten aus den Plasmidklonen stammen, oder Oligonukleotide, die aus der Sequenzierung der Plasmidklone stammen, die auf der Oberfläche des festen Trägers in vordefinierten Orten oder Regionen immobilisiert sind.

## Revendications

1. Procédé d'identification de gènes indispensables à la croissance d'organismes unicellulaires incluant les bactéries, les virus, et les champignons, comprenant :
(a) préparer une banque génomique de l'organisme unicellulaire ;
(b) produire une pluralité de microréseaux identiques, chaque microréseau comportant une surface sur laquelle une pluralité d'éléments provenant de la banque génomique définie dans (a) est immobilisée au niveau de zones prédéfinies sur ladite surface ;
(c) soumettre l'organisme unicellulaire à mutagenèse par transfection à l'aide *(i)* d'un transposon s'intégrant au hasard ou *(ii)* d'un élément transposable ou insertionnel similaire de séquence connue ou (iii) d'un vecteur suicide chimère ;
(d) faire croître une culture test contenant l'organisme unicellulaire ayant subi la mutagenèse et une culture témoin contenant des organismes unicellulaires n'ayant pas subi la mutagenèse dans un ensemble de conditions définies ;
(e) récolter les cellules survivantes des cultures ;
(f) extraire et isoler l'ADN des cellules récoltées de la culture test ;
(g) extraire et isoler l'ARN ou l'ADN des cellules récoltées de la culture témoin ;
(h) produire des sondes de polynucléotides marquées en utilisant une réaction d'élongation d'amorce oligonucléotidique dirigée contre (i) le transposon s'intégrant au hasard ou (ii) l'élément transposable ou insertionnel similaire de séquence connue ou (iii) le vecteur suicide chimère, où la réaction se prolonge dans l'ADN génomique flanquant ;
(i) produire des sondes de polynucléotides marquées à partir de l'ARN ou de l'ADN isolé de la culture témoin ;
(j) hybrider les sondes marquées produites à partir de l'ADN isolé de la culture test avec un premier microréseau identique pour produire un profil d'hybridation test ;
(k) hybrider les sondes marquées produites à partir de l'ARN ou de l'ADN isolé de la culture témoin avec un second microréseau identique pour produire un profil d'hybridation témoin ;
(l) comparer les profils d'hybridation afin d'identifier les gènes indispensables à la croissance dans l'organisme unicellulaire ; et
(m) confirmer que ledit gène identifié est indispensable à la croissance de l'organisme unicellulaire.

2. Procédé de la revendication 1, dans lequel l'ensemble de conditions définies de l'étape (d) comporte des conditions de culture *in vitro* standard non pathogènes pour l'organisme unicellulaire.

3. Procédé de la revendication 1, dans lequel l'ensemble de conditions définies de l'étape (d) comporte des conditions *in vitro* qui reflètent ou imitent des conditions *in vivo*, choisies dans le groupe formé par : des conditions aérobies ou anaérobies, des conditions auxotrophes, de choc thermique, de choc osmotique, l'adjonction ou la présence d'antibiotiques ou de substances pharmaceutiques, des variations de sources de carbone, et des conditions pathogènes *in vivo.*

4. Procédé de la revendication 1, dans lequel la récolte des cellules survivantes de l'étape (e) s'effectue (i) au cours de la croissance exponentielle précoce, (ii) au cours de la croissance exponentielle tardive, (iii) au cours de la croissance en phase stationnaire, ou (iv) au cours de la croissance en phase stationnaire tardive.

5. Procédé de la revendication 1, dans lequel :
l'étape (d) comporte par ailleurs la culture d'autres cultures test et témoin dans un ensemble différent de conditions définies ; et
l'étape (1) comporte la comparaison des profils d'hybridation test et témoin à partir des cellules cultivées dans les différents ensembles de conditions définies.

6. Procédé de la revendication 1, dans lequel les gènes indispensables à l'organisme choisi sont identifiés en déterminant (i) des profils d'hybridation identiques pour la totalité des cellules cultivées dans les différents ensembles de conditions définies, ou (ii) des différences entre les profils d'hybridation test et témoin pour les cellules cultivées dans les différents ensembles de conditions définies.

7. Procédé d'identification de gènes indispensables à la croissance d'organismes unicellulaires incluant les bactéries, les virus et les champignons par identification de gènes mutants létaux conditionnels, comprenant :
(a) préparer une banque génomique de l'organisme unicellulaire : (i) dans un vecteur d'intégration ; ou (ii) dans un vecteur d'expression ;
(b) produire un microréseau comportant une surface sur laquelle une pluralité d'éléments provenant de la banque génomique définie dans (a) est immobilisée au niveau de zones prédéfinies sur ladite surface ;
(c) soumettre l'organisme unicellulaire à mutagenèse par transfection à l'aide (i) d'un transposon s'intégrant au hasard ou (ii) d'un élément transposable ou insertionnel similaire de séquence connue ou (iii) d'un vecteur suicide chimère ;
(d) faire croître les organismes unicellulaires ayant subi la mutagenèse dans des conditions permissives et non permissives pour identifier des organismes unicellulaires ayant subi la mutagenèse, contenant des gènes mutants létaux conditionnels, afin de fournir un pool de gènes mutants létaux conditionnels ;
(e) transformer le pool d'organismes unicellulaires contenant lesdits gènes mutants létaux conditionnels avec la banque génomique de l'étape (a) ;
(f) faire croître les cellules transformées dans les mêmes conditions non permissives qu'à l'étape (d) afin d'identifier les cellules transformées dans lesquelles les gènes mutants létaux conditionnels ont été complétés par le vecteur plus des séquences génomiques complémentaires ;
(g) récolter les cellules survivantes ;
(h) extraire et isoler l'ADN des cellules récoltées ;
(i) produire des sondes de polynucléotides marquées en utilisant une réaction d'élongation d'amorce oligonucléotidique dirigée contre (i) le transposon s'intégrant au hasard ou (ii) l'élément transposable ou insertionnel similaire de séquence connue ou (iii) le vecteur suicide chimère, où la réaction se prolonge dans l'ADN génomique flanquant ; et
(j) hybrider les sondes marquées produites à partir de l'ADN isolé avec un microréseau selon l'étape (b), manière dont les sondes qui s'hybrident avec le microréseau identifient des gènes indispensables à la croissance de l'organisme unicellulaire.

8. Procédé de la revendication 1 ou 7, dans lequel la pluralité d'éléments provenant de la banque génomique de l'étape (b) comprend des bactéries contenant des clones individuels appliquées sous forme de gouttes et cultivées sur une surface du support solide au niveau de positions ou de zones prédéfinies, ou des clones plasmidiques isolés à partir de ladite banque, des produits de PCR provenant des inserts issus des clones plasmidiques, ou des oligonucléotides provenant du séquençage des clones plasmidiques, qui sont immobilisés sur la surface du support solide au niveau de positions ou de zones prédéfinies.


### SEQUENCE LISTING

(1) GENERAL INFORMATION
   (i) APPLICANT: SMITHKLINE BEECHAM CORPORATION
   (ii) TITLE OF THE INVENTION: Methods for Identifying Genes Essential to the Growth of an Organism
   (iii) NUMBER OF SEQUENCES: 4
   (iv) CORRESPONDENCE ADDRESS:
      (A) ADDRESSEE: SmithKline Beecham Corporation
      (B) STREET: 709 Swedeland Road
      (C) CITY: King of Prussia
      (D) STATE: PA
      (E) COUNTRY: USA
      (F) ZIP: 19046
   (v) COMPUTER READABLE FORM:
      (A) MEDIUM TYPE: Diskette
      (B) COMPUTER: IBM Compatible
      (C) OPERATING SYSTEM: DOS
      (D) SOFTWARE: FastSEQ for Windows Version 2.0
   (vi) CURRENT APPLICATION DATA:
      (A) APPLICATION NUMBER: Unknown
      (B) FILING DATE:
      (C) CLASSIFICATION:
   (vii) PRIOR APPLICATION DATA:
      (A) APPLICATION NUMBER: 60/030,159
      (B) FILING DATE: 06-NOV-1996
   (viii) ATTORNEY/AGENT INFORMATION:
      (A) NAME: Gimmi, Edward R
      (B) REGISTRATION NUMBER: 38,891
      (C) REFERENCE/DOCKET NUMBER: P50572
   (ix) TELECOMMUNICATION INFORMATION:
      (A) TELEPHONE: 610-270-4478
      (B) TELEFAX: 610-270-5090
      (C) TELEX:
(2) INFORMATION FOR SEQ ID NO:1:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (xi) SEQUENCE DESCRIPTION: SEQ ID NO:1:
      AATTAACCCT CACTAAAGGG AACA 24
(2) INFORMATION FOR SEQ ID NO:2:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (xi) SEQUENCE DESCRIPTION: SEQ ID NO:2:
      TGTTCCCTTT AGTGAGGGTT AATT 24
(2) INFORMATION FOR SEQ ID NO:3:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (xi) SEQUENCE DESCRIPTION: SEQ ID NO:3:
      GTAATACGAC TCACGGAGGG GCGA 24
(2) INFORMATION FOR SEQ ID NO:4:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:4:
      ACGCCCCTCC GTGAGTCGTA TTAG 24